# EUROPÄISCHE PATENTANMELDUNG

(11) **EP 1 205 511 A1**
(43) Veröffentlichungstag der Anmeldung: **15.05.2002**
(21) Anmeldenummer: 01124647.7
(22) Anmeldetag: 16.10.2001
(51) Int. Cl.: C08J 9/14, C08J 9/42, C08J 9/36

(54) **Flexible, offenzellige, mikrozelluläre Polymerschäume**

(30) Priorität: 07.11.2000 DE 10055084
(71) Anmelder: BASF AKTIENGESELLSCHAFT, 67056 Ludwigshafen (DE)
(72) Erfinder: Dietzen, Franz-Josef, Dr., 67454 Hassloch (DE); Hähnle, Hans-Joachim, Dr., 67435 Neustadt (DE); Evsioukov, Serguei, Dr., 67069 Ludwigshafen (DE); Schornick, Gunnar, Dr., 67271 Neuleiningen (DE); Ehrmann, Gerd, Dr., 67146 Deidesheim (DE); Funk, Rüdiger, Dr., 65527 Niedernhausen (DE)

(57) **Zusammenfassung**

Die Erfindung betrifft flexible, offenzellige, mikrozelluläre Schäume auf Basis von thermoplastischen Polymeren, sowie die Verwendung derartiger Schäume für flüssigkeitsabsorbierende Hygieneartikel.

## Beschreibung

Die Erfindung betrifft flexible, offenzellige, mikrozelluläre Polymerschäume, sowie die Verwendung derartiger Schäume zur Herstellung von flüssigkeitsabsorbierenden Hygieneartikeln. Für das zuverlässige Funktionieren von Hygieneartikeln wie Babywindeln, Inkontinenzprodukten, Artikeln für Damenhygiene oder auch Wundauflagen und Verbänden ist es notwendig, daß drei Aufgaben zuverlässig gelöst werden:
- Aufnahme der Körperflüssigkeit (Akquisition)
- Gleichmäßige Verteilung der aufgenommenen Flüssigkeit (Distribution)
- Druckfeste Speicherung der Flüssigkeit (Storage).

Dabei wird die letzte Aufgabe in den bestehenden Hygieneartikeln in der Regel mit Hilfe von superabsorbierenden Polymeren zuverlässig gelöst. Dagegen bedingt der beständige Trend zu dünneren Hygieneartikeln, daß die bisher zur Bewältigung von Aufnahme und Verteilung verwendeten Materialien (häufig Cellulose Fluff) durch andere Materialien ersetzt werden müssen. Als besonders interessante Materialien haben sich offenzellige Schäume herausgestellt. Zusätzlich zur Offenzelligkeit müssen Sie weich und flexibel sein und eine hydrophile Oberfläche aufweisen um eine rasche Benetzung durch die wässrigen Körperflüssigkeiten gewährleisten zu können.

Es ist bekannt, z.B. aus WO 99/26670, daß derartige Schäume mit einer Zellgröße von 0,01 bis 0,1 mm aus wasserlöslichen Polymeren, z.B. Carboxymethylcellulose, Natriumpolyacrylat oder Polyethylenoxid hergestellt werden können durch Gefriertrocknung der wässrigen Polymerlösung und Erwärmen der Masse unter Schaumbildung. Dies ist jedoch ein extrem aufwendiger Prozeß, der verfahrenstechnisch nur schwer zu kontrollieren ist.

Nach WO 93/04113 werden Polyurethanschäume zur Herstellung von Hygieneartikeln mit wässrigen Salzlösungen behandelt, um eine hydrophile Oberflächenschicht zu bilden, die z.B. Calciumchlorid enthält. Auch dieses Verfahren ist recht aufwendig, da mit großen Mengen Wasser hantiert werden muß, die dann wieder verdampft werden müssen.

In DE-A 43 25 879 wird die Herstellung von offenzelligen Schaumfolien, u.a. auch auf der Basis von Polyolefinen, durch Schaumextrusion beschrieben. Die Offenzelligkeit wird durch Zugabe von Fremdpolymeren bei der Extrusion und durch Erhöhung der Schmelzetemperatur erreicht. Die Zellgröße soll kleiner als 1 mm, insbesondere kleiner als 0,4 mm sein. Es findet sich aber keine Angabe darüber, wie Zellen erzeugt werden können, deren mittlerer Durchmesser kleiner als 0,1 mm ist. In den Beispielen wird ein Polystyrolschaum mit Hilfe von Butan oder einem halogenierten Kohlenwasserstoff als Treibmittel hergestellt. Die Schäume können im Verpackungs- und Bausektor oder als Isoliermaterialien verwendet werden, es findet sich kein Hinweis auf eine Verwendung als Hygieneartikel.

In US 5 098 782 und US 5 387 050 ist die Herstellung offenzelliger Schäume durch Schaumextrusion einer Mischung von LDPE und einem anderen Polymeren, z.B. EVA beschrieben. Über Zellgröße und eine Verwendung als Hygieneartikel werden keine Angaben gemacht.

In US 5 348 795 wird die Herstellung von offenzelligen Polypropylenschäumen für Polster- und Verpackungszwecke beschrieben. Die Offenzelligkeit wird durch Anpassen der Schäumtemperatur erreicht. In den Beispielen sind Zellgrößen im Bereich von 0,1 bis 2,0 mm angegeben.

In WO 96/00258 sind offenzellige Polystyrol-Schäume als Dachisoliermaterialien beschrieben. Die mittlere Zellgröße kann zwischen 0,08 und 1,2 mm liegen.

Nach WO 96/34038 können Vakuum-Isolierplatten aus Polystyrolschäumen mit einem mittleren Zelldurchmesser von kleiner als 0,07 mm hergestellt werden.

Schließlich beschreibt EP-A 754 632 Verpackungsschalen aus Polystyrol-Schaumfolien mit einem Zelldurchmesser zwischen 0,1 und 1,5 mm.

Der Erfindung lag also die Aufgabe zugrunde, flexible, offenzellige, mikrozelluläre Schäume auf Basis von Olefinpolymerisaten oder thermoplastischen Polyestern mit einem mittleren Zelldurchmesser von weniger als 0,1 mm bereitzustellen. Eine weitere Aufgabe war die Entwicklung von flexiblen, offenzelligen Schaumstoffen, die für flüssigkeitsaufnehmende Hygieneartikel geeignet sind und sich nach einfachen Verfahren herstellen lassen.

Ein Gegenstand der Erfindung sind demgemäß flexible, offenzellige, mikrozelluläre Schäume auf Basis von Olefinpolymerisaten oder thermoplastischen Polyestern mit folgenden Eigenschaften:
- eine Offenzelligkeit von mehr als 40 %, vorzugsweise mehr als 50 %,
- eine Dichte von weniger als 300 g/l, vorzugsweise von 20 bis 200 g/l, und
- ein mittlerer Zelldurchmesser von weniger als 0,1 mm, vorzugsweise weniger als 0,08 mm.

Damit flexible Schäume erhalten werden, muß man von entsprechenden Polymeren ausgehen.

Geeignet sind Olefinpolymerisate, wie Polyethylen und Polypropylen, sowie Copolymere des Ethylens und Propylens miteinander und mit untergeordneten Mengen Vinylacetat, Butadien, Acrylester oder Acrylsäure. Besonders bevorzugt ist ein Copolymer aus Ethylen und 5 bis 30 Gew.-% Vinylacetat (EVA). Ferner sind thermoplastische Polyester geeignet, wie Polyethylenterephthalat, Polybutylenterephthalat, Polyethylenglykolterephthalat und ein biologisch abbaubarer Copolyester aus Terephthalsäure, Adipinsäure und Butandiol. Geeignet sind auch Mischungen der genannten Polymeren.

Zur Herstellung der erfindungsgemäßen Schäume werden die Polymeren in einem Extruder aufgeschmolzen, dann wird ein flüchtiges Treibmittel in den Extruder eingepreßt. Damit ausreichend feinzellige Schäume erhalten werden, müssen stark nucleierend wirkende Treibmittel in einer Menge von 2 bis 40 Gew.-%, vorzugsweise 6 bis 20 Gew.-%, bezogen auf das Polymere, verwendet werden. Geeignet sind z.B. Tetrafluorethan (HFKW 134a), Trifluorethan (HFKW 143a) und Kohlendioxid, daneben auch Schwefelhexafluorid, Stickstoff, Argon und Helium, sowie Mischungen dieser Gase. Zusätzlich können noch schwach nucleierend wirkende Treibmittel in Mengen von 2 bis 40 Gew.-%, bezogen auf das Polymere zugesetzt werden, z.B. Kohlenwasserstoffe, Alkohole, Ether, Wasser und Halogenkohlenwasserstoffe, wie Difluorethan (HFKW 152a) und Monochlordifluorethan (HFCKW 142b).

Der Schmelze können ferner die üblichen Zusatzstoffe, wie Keimbildner, z.B. Talkum, Flammschutzmittel, Farbmittel, Antistatika und Stabilisatoren zugesetzt werden.

Damit die erforderliche Offenzelligkeit erhalten wird, wird die Extrusion bei verhältnismäßig hoher Temperatur durchgeführt und/ oder es wird eine Kombination von stark und schwach nucleierenden Treibmitteln verwendet, beispielsweise Kombinationen von Tetrafluorethan (134a) mit Difluorethan (152a) oder Monochlordifluorethan (142b) oder von CO₂ mit Difluorethan.

Ein weiterer Gegenstand der Erfindung ist die Verwendung von flexiblen, offenzelligen, mikrozellulären Extruder-Schäumen auf Basis von thermoplastischen Polymeren zur Herstellung von flüssigkeitsabsorbierenden Hygieneartikeln.

Hierfür sind außer den oben genannten Thermoplasten auch flexibisierte Styrolpolymerisate geeignet, z.B. Styrol/Butadien-Copolymere und mit Polybutadien- oder Polyacrylat-Kautschuk schlagzäh modifiziertes Polystyrol oder Styrol/Acrylnitril-Copolymerisate.

Diese Thermoplasten werden wie oben beschrieben in der Schmelze mit Treibmitteln gemischt und extrudiert.

Die erhaltenen Schäume weisen vorzugsweise folgende Eigenschaften auf:
- eine Offenzelligkeit von mehr als 40 %, vorzugsweise von mehr als 50 %,
- eine Dichte von weniger als 300 g/l, vorzugsweise von 20 bis 200 g/l,
- einen mittleren Zelldurchmesser von weniger als 0,5 mm, vorzugsweise weniger als 0,2 mm und insbesondere weniger als 0,1 mm.

Für die Anwendung als Hygieneartikel sollten die Schäume, vorzugsweise oberflächlich hydrophiliert sein. Die Hydrophilie der Schäume kann durch Verwendung hydrophiler (Co)Polymeren, z.B. Acrylsäure, als Ausgangsmaterialien oder durch Zusatzstoffe während der Schaumherstellung, wie z.B. durch Zusatz von Tensiden und/oder hydrophilen (Co)Polymeren erreicht werden. Bevorzugt werden jedoch Verfahren, bei dem nachträglich eine hydrophile Schicht an der Schaumoberfläche erzeugt wird. Dafür stehen unterschiedliche Möglichkeiten zur Verfügung:
- Durch Behandlung mit Coronaentladung, Plasmabehandlung oder durch die oberflächliche Anwendung von Ozon.
- Durch oberflächliche Verseifung der verwendeten thermoplastischen Copolymere z.B. durch Verseifung des Vinylacetatanteils in den EVA-Polymeren.
- Durch die Adsorption an der Schaumoberfläche von hydrophilierenden Komponenten, wie z.B. Tensiden oder hydrophilen Polymeren, die gegebenenfalls eine hydrophobe Modifikation aufweisen.
- Durch chemische Anbindung von hydrophilen Reagenzien auf der Oberfläche, z.B. Polyamine, wie Polyvinylamin oder Polyethylenimin; Polyepoxide, Polycarbonsäuren, wie Polyacrylsäure.
- Durch Aufbringen einer vernetzten, hydrophilen Hülle entweder durch Reagenzien, die mit sich selbst ein Netzwerk bilden können, z.B. von Additionsprodukten von Epichlorhydrin an Amidoamine.
- Durch den Auftrag von Monomeren oder Polymeren, die mit einem zugesetzten Vernetzer reagieren können, z.B. Polycarbonsäuren in Kombination mit multifunktionellen Epoxiden oder Polyaminen; Polyamine in Kombination mit multifunktionellen Epoxiden, wie Polyvinylimin oder Polyethylenimin mit einem Bisglycidylether eines Oligoethylenglykols, ferner Acrylaten oder Estern.

Die in den Beispielen genannten Prozente beziehen sich auf das Gewicht.

### Beispiel 1

Die Schaumstoffproben wurden durch Extrusion auf einer Tandemanlage hergestellt. Diese besteht aus einem Doppelschneckenextruder ZSK 30 und einem Einschneckenkühlextruder. Polymer und Zusatzstoffe werden der ZSK 30 (Schneckendurchmesser 30 mm) zugeführt. Das Polymere wird aufgeschmolzen und die Treibmittel werden eingemischt. Die treibmittelhaltige Schmelze wird dann im zweiten Extruder (Schneckendurchmesser 60 mm) auf die zum Schäumen notwendige Temperatur abgekühlt.

Der Durchsatz betrug 10 kg/h, die Düse hatte einen Durchmesser von 1,5 mm. Als Treibmittel wurden Mischungen von Monochlordifluorethan (HFCKW 142b) und Tetrafluorethan (HFKW 134a) eingesetzt. Bei dem Polymer handelte es sich um das Ethylen-Vinylacetat Copolymer (EVA) EVATANE 1040 (VA Anteil: 14 %; MFI: 4) der Firma Elf-Atochem. Als Keimbildner wurde 1,0 % Talkum zugegeben.

Die Ergebnisse sind in der folgenden Tabelle 1 zusammengefaßt:

**Tabelle 1**

| Versuch | 142b(%) | 134a (%) | Schmelzetemp. (°C) | Dichte (g/l) | Offenzelligkeit (%) | Zellgröße (mm) |
|---|---|---|---|---|---|---|
| 1 | 30 | 0 | 80,5 | 39 | 3 | 0,300 |
| 2 | 25 | 5 | 76,2 | 44 | 4 | 0,150 |
| 3 | 20 | 10 | 86,1 | 114 | 76 | 0,040 |
| 4 | 20 | 10 | 85,6 | 154 | 69 | 0,030 |
| 5 | 15 | 10 | 86,3 | 149 | 63 | 0,025 |
| 6 | 15 | 10 | 83,5 | 168 | 60 | 0,020 |

### Beispiel 2

Beispiel 1 wurde wiederholt, wobei aber als Polymer ein Copolyester aus Terephthalsäure, Adipinsäure und Butandiol (ELCOFLEX der BASF AG) eingesetzt wurde. Ergebnisse siehe Tabelle 2.

**Tabelle 2**

| Versuch | 142b(%) | 134a (%) | Schmelzetemp. (°C) | Dichte (g/l) | Offenzelligkeit (%) | Zellgröße (mm) |
|---|---|---|---|---|---|---|
| 1 | 12,5 | 0 | 95,6 | 39 | 4 | 0,15 |
| 2 | 7,5 | 5 | 96,3 | 178 | 49 | 0,09 |
| 3 | 5 | 7,5 | 93,0 | 91 | 61 | 0,08 |
| 4 | 2,5 | 10 | 93,4 | 107 | 73 | 0,07 |

### Beispiel 3

Aus dem nach Beispiel 1, Versuch 3 hergestellten EVA-Schaum und aus dem nach Beispiel 2, Versuch 3 hergestellten ECOFLEX-Schaum wurden mit einem scharfen Messer Stücke mit den Abmessungen 0,3 x 5 cm herausgeschnitten. Diese wurden in jeweils 1 %ige Lösungen von diversen Reagenzien (s. Tabelle 2) in iso-Propanol eingetaucht und 3 mal kurz evakuiert (bis keine Bläschen mehr entweichen). Dann wurden die Schaumstückchen herausgeholt, auf Filterpapier gelegt, um überschüssige Lösung zu entfernen und anschließend im Trockenschrank bei 40°C unter Vakuum getrocknet. Die resultierende Hydrophilie wurde durch Aufbringen von Wassertropfen auf die Oberfläche den Schäumen beurteilt. Bei den meisten behandelten Proben war eine sehr schnelle Wasseraufnahme zu beobachten (Tabelle 3). Diese Hydrophilie bleibt sogar nach einmaliger Wäsche mit reinem Wasser erhalten.

**Tabelle 3**

| Nr. | Tensid (1 % Lösung in iso-PrOH) | Wasseraufnahme | | |
|---|---|---|---|---|
| | | ECOFLEX | EVA | |
| | | | behandelt | gewaschen |
| 1 | keine | - | - | - |
| 2 | Nuwet 100 (Fa. OSI) | ++ | ++ | + |
| 3 | Nuwet 300 (") | + | + | - |
| 4 | Nuwet 500 Finish (") | ++ | + | + |
| 5 | Cremophor WOCE 5115 (Fa. BASF) | ++ | + | +/- |
| 6 | Cremophor A6 (Fa. BASF) | ++ | +/- | - |
| 7 | Lutensol LF 400 (Fa. BASF) | ++ | ++ | ++ |
| ++ sehr schnell; | | | | |
| + schnell; | | | | |
| +/- langsam; | | | | |
| - keine Aufnahme, das Tropfen bleibt auf der Oberfläche liegen. | | | | |

### Beispiel 4

Aus den nach Beispiel 1, Versuch 3 hergestellte EVA-Schaum und dem nach Beispiel 2, Versuch 3 hergestellten ECOFLEX-Schaum wurden mit einem scharfen Messer Stücke mit den Abmessungen 0,3 x 5 cm abgeschnitten. Diese wurde in eine 1 %ige Lösung von Polyvinylamin (K-Wert 90) in einer 1:1-Mischung aus Wasser/iso-Propanol getaucht und 3 mal kurz evakuiert. Dann wurden die Schaumstücke aus der Lösung genommen, auf Filterpapier gelegt, um überschüssige Lösung zu entfernen und anschließend bei 40°C im Vakuum getrocknet. Die resultierende Hydrophilie wurde wie bei Beispiel 3 geprüft.

### Beispiel 5

Aus dem nach Beispiel 1, Versuch 3 hergestellte EVA-Schaum und dem nach Beispiel 2, Versuch 3 hergestellten ECOFLEX-Schaum wurden mit einem scharfen Messer Stücke mit den Abmessungen 0,3 x 5 cm abgeschnitten. In einer auf 10°C gekühlten 1:1-Mischung aus Wasser/iso-Propanol wurde eine 1 %ige Lösung aus Polyvinylamin (K-Wert 90) hergestellt und 5 %, bezogen auf das Polyvinylamin, an Ethylenglycolbisglycidylether zugegeben. In diese Reaktionsmischung wurden die Schäume getaucht und 3 mal kurz evakuiert. Dann wurden die Schaumstücke aus der Lösung genommen, auf Filterpapier gelegt, um überschüssige Lösung zu entfernen und anschließend bei 40°C im Vakuum getrocknet. Die resultierende Hydrophilie wurde wie bei Beispiel 3 geprüft.

### Beispiel 6

Aus dem nach Beispiel 1, Versuch 3 hergestellte EVA-Schaum und dem nach Beispiel 2, Versuch 3 hergestellten ECOFLEX-Schaum wurden mit einem scharfen Messer Stücke mit den Abmessungen 0,3 x 5 cm abgeschnitten. Diese wurden in eine 1 %ige Lösung von Polyvinylamin (K-Wert 90) in einer 1:1-Mischung aus Wasser/iso-Propanol getaucht und 3 mal kurz evakuiert. Dann wurden die Schaumstücke aus der Lösung genommen, auf Filterpapier gelegt, um überschüssige Lösung zu entfernen und anschließend bei 40°C im Vakuum getrocknet. Anschließend wurde die erhaltene Probe in eine 1 %ige Lösung von Polyacrylsäure (K-Wert 110) in einer 1:1-Mischung aus Wasser/iso-Propanol getaucht und 3 mal kurz evakuiert. Dann wurden die Schaumstücke aus der Lösung genommen, auf Filterpapier gelegt, um überschüssige Lösung zu entfernen und anschließend bei 40°C im Vakuum getrocknet. Die resultierende Hydrophilie wurde wie bei Beispiel 3 geprüft.

**Tabelle 4**

| Beispiel | Wasseraufnahme | | | |
|---|---|---|---|---|
| | Ecoflex | | EVA | |
| | Behandelt | Gewaschen | Behandelt | Gewaschen |
| 4 | ++ | + | ++ | + |
| 5 | ++ | ++ | ++ | ++ |
| 6 | ++ | ++ | ++ | ++ |

## Patentansprüche

1. Flexible, offenzellige, mikrozelluläre Schäume auf Basis von Olefinpolymerisaten oder thermoplastischen Polyestern, **dadurch gekennzeichnet, daß** sie aufweisen:
• eine Offenzelligkeit von mehr als 40 %,
• eine Dichte von weniger als 300 g/l und
• einen mittleren Zelldurchmesser von weniger als 0,1 mm.

2. Verfahren zur Herstellung der Schäume nach Anspruch 1 durch Extrusion der Polymerschmelze zusammen mit 2 bis 40 Gew.-%, bezogen auf das Polymere, eines oder mehrerer stark nucleierend wirkender, flüchtiger Treibmittel.

3. Verfahren nach Anspruch 2, **dadurch gekennzeichnet, daß** die stark nucleierend wirkenden Treibmittel Tetrafluorethan, Trifluorethan, Kohlendioxid, Schwefelhexafluorid, Stickstoff, Argon und/oder Helium sind.

4. Verfahren nach Anspruch 2, **dadurch gekennzeichnet, daß** zusätzlich zu den stark nucleierenden Treibmitteln auch noch schwach nucleierende Treibmittel in Mengen von 2 bis 40 Gew.-%, bezogen auf das Polymere, eingesetzt werden.

5. Verwendung von flexiblen, offenzelligen, mikrozellulären Extruder-Schäumen auf Basis von thermoplastischen Polymeren zur Herstellung von flüssigkeitsabsorbierenden Hygieneartikeln.

6. Verwendung nach Anspruch 5, **dadurch gekennzeichnet, daß** die Schäume folgende Eigenschaften aufweisen:
• eine Offenzelligkeit von mehr als 40 %,
• eine Dichte von weniger als 300 g/l und
• einen mittleren Zelldurchmesser von weniger als 0,5 mm.

7. Verwendung nach Anspruch 5, **dadurch gekennzeichnet, daß** die Schäume hydrophiliert sind.

8. Verwendung nach Anspruch 7, **dadurch gekennzeichnet, daß** die Hydrophilierung durch Behandeln der Schaumoberfläche mit einem Tensid erfolgte.

9. Verwendung nach Anspruch 7, **dadurch gekennzeichnet, daß** die Hydrophilierung durch Behandeln der Schaumoberfläche mit einem Polyamin oder einer Polycarbonsäure erfolgte.

10. Verwendung nach Anspruch 7, **dadurch gekennzeichnet, daß** die Hydrophilierung durch Aufbringen einer vernetzten Hülle auf die Schaumoberfläche erfolgte, wobei die Hülle aus einem hydrophilen Polymer und einem Vernetzer hergestellt wurde.
